(19) Europäisches Patentamt
European Patent Office
Office européen des brevets

(11) Publication number: 0 099 194
A2

(12) EUROPEAN PATENT APPLICATION

(21) Application number: 83303553.8

(22) Date of filing: 21.06.83

(51) Int. Cl.³: C 07 D 451/04
C 07 D 451/06, C 07 D 451/14
A 61 K 31/46, A 61 K 31/435

(30) Priority: 03.07.82 GB 8219297

(43) Date of publication of application:
25.01.84 Bulletin 84/4

(84) Designated Contracting States:
BE CH DE FR GB IT LI NL SE

(71) Applicant: BEECHAM GROUP PLC
Beecham House Great West Road
Brentford Middlesex(GB)

(72) Inventor: Hadley, Michael Stewart
9 Coney Gree
Sawbridgeworth Hertfordshire(GB)

(72) Inventor: Watts, Eric Alfred
217 Waterbouse Moor
Harlow Essex(GB)

(74) Representative: Stott, Michael John et al,
European Patent Attorney Beecham Pharmaceuticals
Great Burgh Yew Tree Bottom Road
Epsom Surrey KT18 5XQ(GB)

(54) Novel benzamides.

(57) Compounds of formula (I) and pharmaceutically acceptable salts thereof:

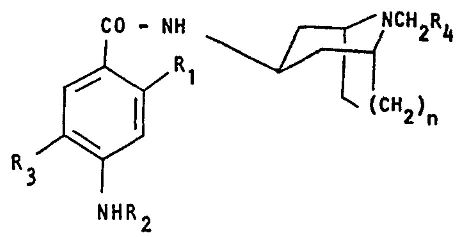

(I)

wherein:

$R_1$ is $C_{1-6}$ alkoxy;
$R_2$ is hydrogen or $C_{1-4}$ alkanoyl;
$R_3$ is chloro or bromo;
$R_4$ is *t*-butyl, *iso*-butyl or cyclopropyl; and
n is 1 or 2;

having anti-depressant and/or anxiolytic activity, a process for their preparation and their use.



Croydon Printing Company Ltd.

NOVEL BENZAMIDES

This invention relates to novel benzamides, to a process for their preparation, to pharmaceutical compositions containing them and their use in the treatment of mammals.

European Patent Application No. 79302978.6 and U.S. Patent No. 4273778 disclose that compounds of the formula (A), and pharmaceutically acceptable salts thereof:

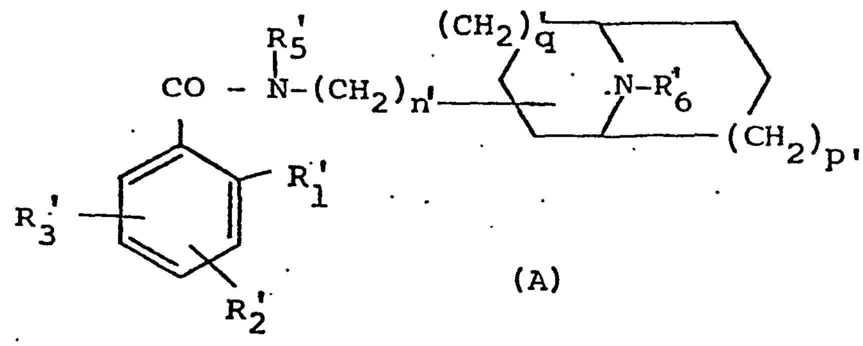

(A)

wherein:

$R_1'$ is a $C_{1-6}$ alkoxy group;

$R_2'$ and $R_3'$ are the same or different and are hydrogen halogen, $CF_3$, $C_{2-7}$ acyl, $C_{2-7}$ acylamino, or amino, aminocarbonyl or aminosulphone optionally substituted by one or two $C_{1-6}$ alkyl groups, $C_{1-6}$ alkylsulphone or nitro;

$R_5'$ is hydrogen or $C_{1-6}$ alkyl;

$R_6'$ is $C_{1-7}$ alkyl or a group $-(CH_2)_s'R_7'$ where s is 0 to 2 and $R_7'$ is a $C_{3-8}$ cycloalkyl group, or a group $-(CH_2)_t'R_8'$ where t is 1 or 2 and $R_8'$ is $C_{2-5}$ alkenyl or a phenyl group optionally substituted by one or two substituents selected from $C_{1-6}$ alkyl, $C_{1-4}$ alkoxy, trifluoromethyl and halogen;

and

$n'$, $p'$ and $q'$ are independently 0 to 2; have useful pharmacological activity. More specifically the compounds of formula (A) are stated to be dopamine antagonists useful in the treatment of disorders related to impaired gastro-intestinal motility and/or in the treatment of psychosis.

The said European Application and US Patent has extensive exemplification of typical compounds of formula (A) and of their dopamine antagonist activity, establishing the veracity of the claimed utilities for the class of compounds defined by formula (A).

We have surprisingly found a hitherto not specifically disclosed small group of compounds having seretonin antagonist activity (anti-5HT activity) such compounds therefore possessing antidepressant and/or anxiolytic activity, coupled with a high specificity of action. These compounds have an advantageous therapeutic ratio (based on dopamine antagonist CNS side effects) over the best and structurally closest compounds specifically disclosed in European Application No. 79302978.6 and U.S. Patent No. 4,273,778.

Accordingly, the present invention provides a compound of formula (I), or a pharmaceutically acceptable salt and/or solvate thereof:

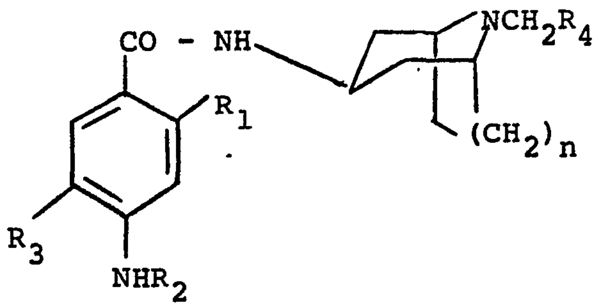

(I)

wherein:

$R_1$ is $C_{1-6}$ alkoxy;
$R_2$ is hydrogen or $C_{1-4}$ alkanoyl;
$R_3$ is chloro or bromo;
$R_4$ is t-butyl, iso-butyl or cyclopropyl; and
n is 1 or 2.

Suitable examples of the group $R_1$ include methoxy ethoxy and n- and iso-propoxy. Preferably $R_1$ is a methoxy group.

Suitable values for the group $R_2$ include hydrogen, formyl, acetyl, propionyl and n- and iso-butyryl. Preferably $R_2$ is hydrogen or acetyl, most preferably hydrogen.

Preferably $R_3$ is chloro.
Preferably $R_4$ is t-butyl.
n is preferably 1.

The pharmaceutically acceptable salts of the compound of the formula (I) include acid addition salts with conventional acids such as hydrochloric, hydrobromic, phosphoric, sulphuric, citric, tartaric, lactic and acetic acid.

The compounds of the formula (I) may also form solvates such as hydrates and the invention extends to such solvates.

A favourable group of compounds within formula (I) is of the formula (II):

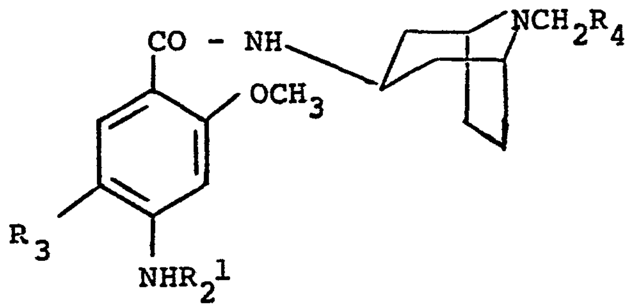

(II)

wherein $R_2^1$ is hydrogen or acetyl and $R_4$ is as defined in formula (I)

$R_2^1$ is preferably hydrogen.
$R_3$ is preferably chloro.
$R_4$ is preferably <u>t</u>-butyl.

A further sub group of compounds within formula (I) is of formula (III):

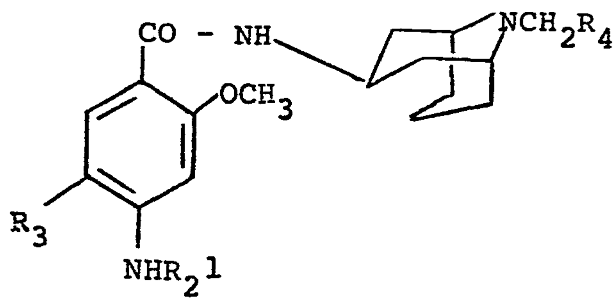

(III)

wherein $R_2^1$ and $R_4$ are as defined in formula (III).
$R_2^1$ is preferably hydrogen.
$R_3$ is preferably chloro.
$R_4$ is preferably <u>t</u>-butyl.

The present invention also provides a process for the preparation of a compound of formula (I) which process comprises the reaction of a compound of the formula (IV):

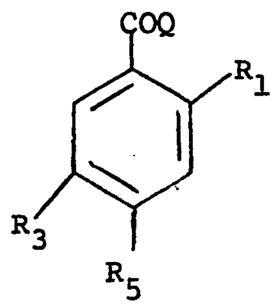

(IV)

with a compound of formula (V):

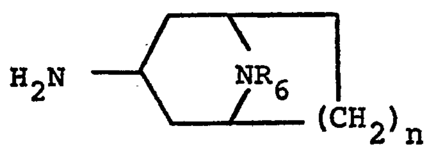

(V)

wherein:

Q is a leaving group;

$R_5$ is amino, protected amino or nitro;

$R_6$ is $CH_2R_4$ as defined or a hydrogenolysable protecting group; and the remaining variables are as defined in formula (I); and thereafter if necessary converting group $R_5$ in the thus formed compound to a group $NHR_2$, converting $R_6$ when other than $CH_2R_4$ and optionally forming a pharmaceutically acceptable salt of the resultant compound of the formula (I).

The leaving group Q is a group readily displaceable by a nucleophile. Suitable examples of Q are hydroxy, halogen such as chloro and bromo and acyloxy such as $C_{1-4}$alkanoyloxy, $C_{1-4}$alkoxycarbonyloxy and activated hydrocarbyloxy such as pentachlorophenoxy.

If the leaving group is hydroxy, then the reaction is preferably carried out in an inert non-hydroxylic solvent, such as benzene, toluene or diethyl ether in the presence of a dehydrating catalyst, such as a carbodiimide, for example dicyclohexylcarbodiimide. The reaction may be carried out at a non-extreme temperature such as - 10 to 100$^{o}$C, for example 0 to 80$^{o}$C.

If the leaving group is halide, then the reaction is is preferably carried out at a non-extreme temperature in an inert non-hydroxylic solvent, such as benzene, dichloromethane toluene or diethyl ether. It is also preferably carried out in the presence of an acid acceptor, such as an organic base, in particular a tertiary amine, such as triethylamine, pyridine or picoline, some of which can also function as the solvent. Alternatively, the acid acceptor can be inorganic, such as calcium carbonate, sodium carbonate or potassium carbonate.

If the leaving group is acyloxy, then the reaction is preferably carried in substantially the same manner as if the leaving group were halide. Suitable examples of acyloxy leaving groups include $C_{1-4}$ alkanoyloxy, mesyloxy, tosyloxy and triflate.

If the leaving group is $C_{1-4}$ alkoxycarbonyloxy, then the reaction is preferably carried out in an inert solvent, such as methylenechloride at a non-extreme temperature in the presence of an acid acceptor, such as triethylamine.

If the leaving group is activated hydrocarbyloxy then the reaction is preferably carried out in an inert polar solvent, such as dimethylformamide. It is also preferred that the activated hydrocarbyloxy group is a pentachlorophenyl ester and that the reaction is carried out at ambient temperature.

Suitable examples of $R_6$ N-protecting groups include benzyl optionally substituted by one or two substituents selected from $C_{1-4}$ alkyl, $C_{1-4}$ alkoxy, trifluoromethyl, halogen and nitro.

The intermediates of the formula (IV) are either known compounds or can be prepared by analogous processes to known compounds.

It will be realised that in the compound of the formula (I) the -CO-NH- linkage has a β orientation with respect to the ring of the bicyclic moiety to which it is attached. A mixture of α and β isomers of the compound of the formula (I) may be synthesised non-stereospecifically and the desired β isomer separated conventionally therefrom, e.g. by chromatography; or alternatively the β isomer may if desired be synthesised from the corresponding β form of the compound of the formula (V).

Synthesis from the corresponding β isomer of the compound of the formula (V) is in general preferred.

The α or β form of the compound of the formula (V) may if desired be prepared by known stereospecific processes, such as those leading to the α and β isomers of the compound of the formula (V) described in Descriptions 3C, 4A and 4C of European Patent Application No. 79302978.6 and U.S. Patent

No. 4273778 for the α-isomers and Descriptions 2 and 3A and B of these Applications for the β-isomers.

The precursor of the compound of the formula (V) may be stereospecifically synthesised, such as the azide (D3) of Description 2 of the above European Application and U.S. Patent, and then converted to the corresponding desired isomer of the compound of the formula (V) under non-stereospecific conditions with retention of configuration. Alternatively, the precursor (such as the oximes and imines of Descriptions 3 and 4 of the above European and US Applications) can be converted under stereospecific conditions to the desired isomer of the compound of the formula (V).

Alternatively a mixture of the α and β isomers of the compound of the formula (V) may be synthesised non-stereospecifically and the desired isomer separated conventionally therefrom e.g. by chromatography. However, in this case it is generally more convenient to react the mixture to give a mixture of α and β isomers of the compound of the formula (I) and to separate these if desired as hereinbefore described.

Compounds of the formula (V) wherein $R_6$ is t-butylmethyl or cyclopropylmethyl are novel and form an aspect of the present invention.

Compounds of formula (IV) wherein $R_5$ is a nitro group and compounds of formula (V) wherein $CH_2R_4$ is replaced by an $R_6$ protecting group are useful intermediates. A number of conversions is possible as follows:

(a) an $R_5$ nitro substituent is convertible to an $R_2$ amino substituent by reduction;

(b) a $C_{1-7}$ acylamino substituent is convertible to an amino substituent by deacylation;

(c) an amino substituent is convertible to a $C_{1-7}$ acylamino substituent by acylation; and

(d) a hydrogen substituent is convertible to a halogen substituent by halogenation.

Conversions (a) to (d) are only exemplary and are not exhaustive of the possibilities.

In regard to (a), the reduction is carried out with a reagent suitable for reducing nitroanisole to aminoanisole.

In regard to (b), deacylation is carried out by treatment with a base, such as an alkali metal hydroxide.

In regard to (c), the acylation is carried out with an acylating agent, such as the corresponding acid or acid chloride. Formylation is carried out with the free acid.

In regard to (d) halogenation is carried out with conventional halogenating agents.

Before carrying out any of these conversions, the effect, if any, on other substituents should be considered and such reagents as are appropriate should be selected together with the adoption of such precautionary measures as are necessary.

Conversion of $R_6$ when a hydrogenolysable protecting group to $CH_2R_4$ may be carried out by deprotection and subsequent reaction with $CH_2R_4L$ wherein L is a leaving group.

L is suitably a group or atom readily displaced by a nucleophile. Suitable values for L include chloride, bromide, iodide, $OSO_2CH_3$ or $OSO_2$. $C_6H_5$.p.$CH_3$.

Favoured values for L include chloro, bromo and iodo.

Deprotection may suitably be effected under conventional conditions. Suitable methods include transition metal catalysed hydrogenation, using for example palladium - or platinum - charcoal at atmospheric pressure or a slight excess thereover. A dry, inert, polar solvent such as dry ethanol at ambient temperature is suitable.

The reaction of the deprotected product with $R_4CH_2L$. may be carried out under conventional alkylation conditions, for example, in an inert solvent such as dimethylformamide in the presence of an acid acceptor such as potassium carbonate. Generally the reaction is carried out at a non-extreme temperature such as at ambient or at a slightly elevated temperature.

In the preparation of the compounds of the invention it is preferred that $R_6$ is $CH_2R_4$ as in the desired compound of formula (I).

It will be appreciated that interconversions of $R_5$ or $R_6$ may take place in any desired or necessary order.

The compounds of the formula (I) are particularly useful in the treatment of depression or anxiety because they combine anti-depressant and/or anxiolytic activity with a good therapeutic ratio (based on dopamine antagonist CNS effects).

In use, the compounds will normally be in a form of a pharmaceutical composition comprising a compound of the formula (I) of a pharmaceutically acceptable salt thereof, together with a pharmaceutically acceptable carrier. These compositions form an important aspect of this invention.

Such pharmaceutical compositions may be adapted for oral or parenteral administration, and as such may be in the form of tablets, capsules, oral liquid preparations, powders, granules, lozenges, reconstitutable powders, injectable and infusable solutions or suspensions; the compositions may also be in the form of suppositories. Normally, orally administrable compositions are preferred.

Tablets and capsules for oral administration may be in unit dose presentation form, and may contain conventional excipients, such as binding agents, fillers, tabletting lubricants, disintegrants and acceptable

wetting agents. The tablets may be coated according to methods well known in normal pharmaceutical practice. Oral liquid preparations may be in the form of, for example aqueous or oily suspensions, solutions, emulsions, syrups or elixirs, or may be presented in a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising the compound of the formula (I) or a pharmaceutically acceptable salt thereof and a sterile vehicle. The compound, depending on the vehicle and concentration used, can be either suspended or dissolved in the vehicle. In preparing solutions the compound can be dissolved for injection and filter sterilised before filling into a suitable vial or ampoule and sealing. Advantageously, adjuvants such as a local anaesthetic, preservatives and buffering agents can be dissolved in the vehicle. Parenteral suspensions are prepared in substantially the same manner, except that the compound is suspended in the vehicle instead of being dissolved and sterilised by exposure to ethylene oxide before suspending in the sterile vehicle. Advantageously, a surfactant or wetting agent is included in the composition to facilitate uniform distribution of the compound.

As is common practice, the compositions will usually be accompanied by written or printed directions for use in the medical treatment concerned.

The dose of the compound used in the treatment of CNS disorders, such as depression or anxiety will vary in the usual way with the seriousness of the disorders, the weight of the sufferer, and other similar factors. However, as a general guide suitable unit doses may be 0.1 to 100 mgs, more suitably 0.1 to 20 mgs, for example 0.5 to 10mgs; and such unit doses may be administered more than once a day, for example two or three times a day, so that the total daily dosage is in the range of about 0.01 to 10 mg/kg- and such therapy may extend for a number of weeks or months.

The invention also provides a method of treatment of CNS disorders related to depression or anxiety in mammals including humans, which comprises administering to the sufferer an effective amount of a compound of the invention, or of a pharmaceutically acceptable salt thereof.

The invention further provides a compound of the invention or a pharmaceutically acceptable salt thereof for use in the treatment of CNS disorders related to depression or anxiety.

The following Examples illustrate the preparation of the compounds of the invention.

The following Descriptions illustrate the preparation of intermediates to the compounds of the present invention.

Description 1

8-Neopentyl-8-azabicyclo(3,2,1)octan-3-one (D1)

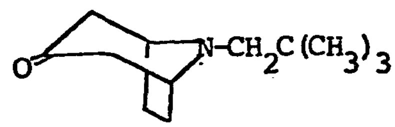

(D1)

Neopentylamine (50g, 0.57mole) was dissolved with cooling in dilute hydrochloric acid (115ml., 5M) and water (300ml). This was added to an aqueous acidic solution of succindialdehyde [prepared from dimethoxytetrahydrofuran (76ml) in water (1L) containing concentrated hydrochloric acid (22ml)], followed by addition of acetone dicarboxylic acid (84g) and tri-sodium orthophosphate (137g) in water (1L).

The mixture (pH 2.5 → 3.0) was stirred at room temperature for 48 hours (pH 7.0). The whole was reacidified to pH 1, extracted with ethyl acetate (3x300ml) and the aqueous layer separated. The acidic aqueous layer was cautiously neutralised with solid potassium carbontate and extracted with ethyl acetate (3x300ml). The combined organic extracts were dried over sodium sulphate, filtered and evaporated in vacuo to yield the title compound (84g, 75%).

Description 2

8-Neopentyl-8-azabicyclo(3,2,1)octan-3-one Oxime (D2)

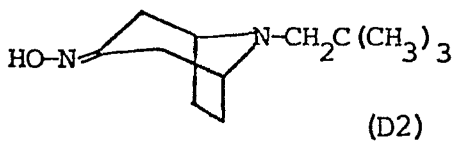

(D2)

The ketone (D1) (84g, 0.43 mole) was dissolved in ethanol (300ml) and treated with hydroxylamine hydrochloride (75g; 2.5 x excess). The whole was warmed to solution on a water bath for one hour then allowed to cool. The oxime hydrochloride was filtered, re-dissolved in water (1L) and neutralised with solid potassium carbonate. The suspension was then extracted with ethyl acetate (3x300 ml) and the organic extracts combined. After drying over potassium carbonate the extracts were filtered and evaporated in vacuo to give the title compound (77.34g, 85%).

Description 3

3-β-Amino-8-neopentyl-8-azabicyclo(3,2,1)octane (D3)

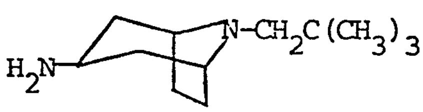

(D3)

The oxime (D2) (77.3g 0.37 mole) was dissolved in amyl alcohol (900ml) and heated to 110$^{o}$ under nitrogen. Sodium (78g) was added in small portions over 3 hrs. at such a rate to maintain reaction temperature between 120$^{o}$ and 140$^{o}$. The whole was maintained at 120$^{o}$ until complete solution.

The mixture was cooled, treated with dilute hydrochloric acid (1L, 5M) and ethyl acetate (1L). The aqueous acidic layer was separated and further washed with ethyl acetate (3x300 ml). After cautious basification with sodium hydroxide the mixture was extracted with ethyl acetate (3 x 300ml). The combined organic extracts were dried over sodium sulphate, filtered and evaporated in vacuo to yield the title compound as an oil (65g) slowly

crystallising. Distillation under nitrogen in vacuo gave the pure product (49.87g, 70%) b.p. 86-88°/0.03mm as a colourless oil/low melting solid.

In a similar manner were prepared:-

Description 4

3-β-Amino-9-neopentyl-9-azabicyclo(3,3,1)nonane (D4)

b.p. 104-106°/0.5mm

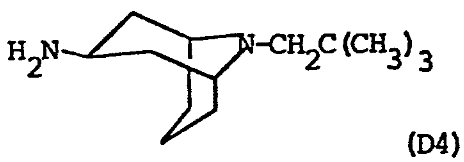

(D4)

Description 5

3-β-Amino-8-cyclopropylmethyl-8-azabicyclo(3,2,1)octane (D5)

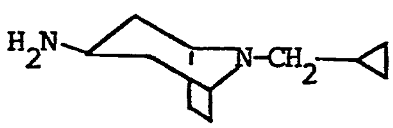

(D5)

Description 6

3-β-Amino-8-isopentyl-8-azabicyclo(3,2,1)octane (D6)

b.p. 80°/0.04mm

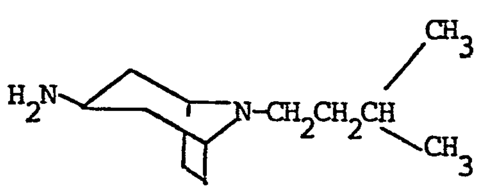

(D6)

Example 1

4-Acetylamino-5-chloro-2-methoxy-N-[3-β-(8-neopentyl-8-azabicyclo (3,2,1)octyl)]benzamide (E1)

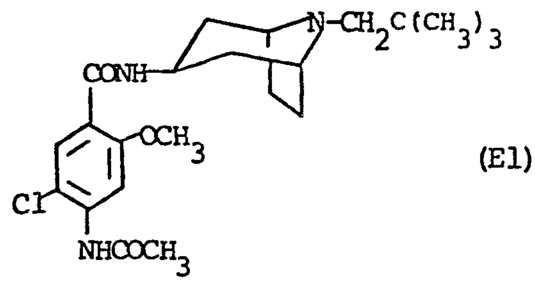

(E1)

4-Acetylamino-5-chloro-2-methoxybenzoic acid (12.43g, 0.051 mole) was suspended in dry methylene chloride (100ml) and treated with oxalyl chloride (4.63ml) and a few drops of dry dimethylformamide. The mixture was stirred at room temperature for 3/4 hr., then the clear solution cooled to $0^{\circ}$.

Triethylamine (22 ml) in methylene chloride (30 ml) was added dropwise at $0^{\circ}$ followed by dropwise addition of 3-β-amino-8-neopentyl-8-azabicyclo (3,2,1) octane (D3) (9.93g, 0.051 mole) in methylene chloride (30 ml). The reaction mixture was allowed to reach ambient temperatures over three hours, then basified with dilute sodium hydroxide (20ml, 2.5M) and the organic layer separated. The aqueous layer was further extracted with chloroform (3 x 100ml). The combined organic extracts were dried over potassium carbonate, filtered and evaporated in vacuo. Subsequent treatment of the residue with dry ether gave 4-acetylamino-5-chloro -2-methoxy-N-[3-β-(8-neopentyl-8-azabicyclo(3,2,1)octyl)]benzamide (16.24g; 75%) as colourless microcrystals. m.p. 203-208°.

$C_{22}H_{32}N_3O_3Cl$

Required % C=62.62; H=7.64; N=9.95; Cl=8.40

Found % C=62.58; H=7.66; N=9.92; Cl=8.69.

Example 2

4-Amino-5-chloro-2-methoxy-N-[3-β-(8-neopentyl-8-azabicyclo (3,2,1)octyl)] benzamide

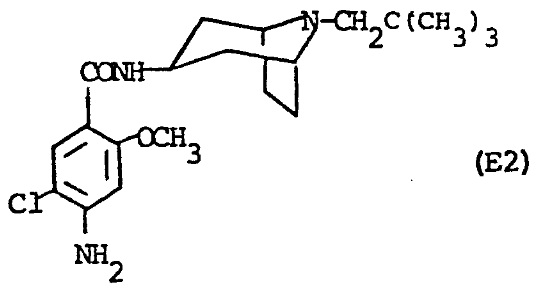

(E2)

4-Acetylamino-5-chloro-2-methoxy-N-[3-β-(8-neopentyl-8-azabicyclo(3,2,1)octyl)]benzamide (E1) (16.24g) on treatment with potassium hydroxide (6g) in ethanol (200ml) and water (50ml) at reflux temperatures for 3/4 hr gave, on cooling, the title compound (11.07g; 91%) as colourless microcrystals mp 250-251°.

$C_{20}H_{30}ClN_3O_2$

Required % C = 63.24; H = 7.91; N = 11.07; Cl = 9.35.

Found % C = 62.83, 62.97; H = 7.83, 7.89; N = 10.85, 10.83; Cl = 8.95, 9.13.

Example 3

4-Amino-5-chloro-2-methoxy-N-[3-β-(8-cyclopropylmethyl-8-azabicyclo (3,2,1)octyl]benzamide

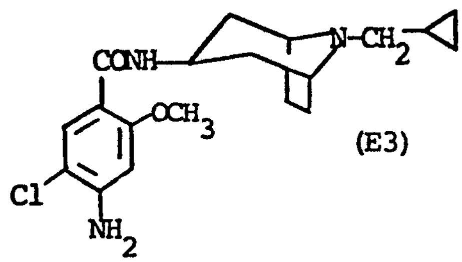

(E3)

4-Acetylamino-5-chloro-2-methoxy benzoic acid (5.28g, 0.022 moles) was suspended in thionyl chloride (50ml) and stirred for ½ hour at room temperature. The solution was evaporated in vacuo, azeotroped twice with anhydrous toluene (100ml) and the resulting acid chloride dissolved in dry methylene chloride (100ml). Treatment with triethylamine (3.0 ml) and 3-β-amino-8-cyclopropylmethyl-8-azabicyclo (3,2,1) octane (D5) (3.6g) in a similar manner to that described in Example 1 followed by similar aqueous ethanolic hydrolysis (E2) gave the title compound (3.86g; 54%) as colourless microcrystals mp 199-200°

$C_{18}H_{24}ClN_3O_2$

Requires % C = 61.80, H = 6.87, N = 12.02

Found % C = 62.10, H = 7.55, 7.50; N = 11.94, 11.84.

Example 4

4-Amino-5-chloro-2-methoxy-N-[3-β-(9-neopentyl-9-azabicyclo (3,3,1)nonyl)]benzamide

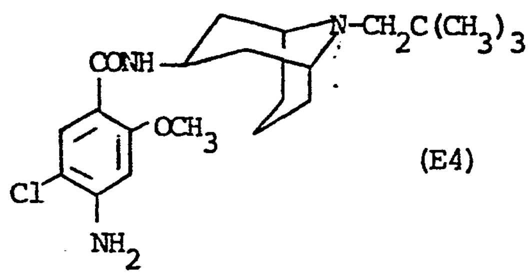

(E4)

4-Acetylamino-5-chloro-2-methoxy benzoic acid (6.57g, 0.027 mole) was treated with oxalyl chloride (2.50ml), triethylamine (11.2 ml) and 3-β-amino-9-neopentyl-9-azabicyclo(3,2,1)nonane (D4) (5.67g, 0.27 mole) in a similar manner to that described in Example 1 followed by aqueous ethanolic hydrolysis to give the title compound (3.4g; 46%) as colourless microcrystals mp 243°.

$C_{21}H_{32}ClN_3O_2$

Required % C = 64.04, H = 8.13, N = 10.67, Cl = 9.02.

Found % C = 63.65, 63.80; H = 8.38, 8.43, N = 10.59, 10.63; Cl = 9.02, 9.17.

Example 5

4-Amino-5-chloro-2-methoxy-N-[3-β-(8-isopentyl-8-azabicyclo(3,2,1)octyl)] benzamide

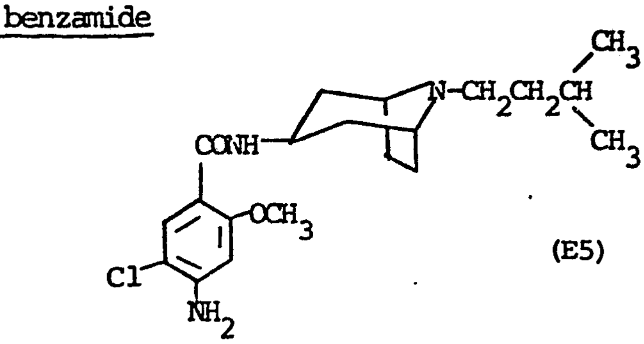

(E5)

4-Acetylamino-5-chloro-2-methoxy benzoic acid (2.0g, 0.0082 mole) thionyl chloride (20ml), triethylamine (1.26ml) and 3-β-amino-8-isopentyl-8-azabicyclo(3,2,1)octane(D6) (1.41g, 0.0082 mole) were reacted in a similar manner to that described in Examples 3 and 1 followed by similar aqueous ethanolic hydrolysis to give the title compound (1.62g;51%) as colourless mp 246-247°

$C_{20}H_{30}ClN_3O_2$

Requires % C = 63.24, H = 7.91, N = 11.07, Cl= 9.35.

Found % C = 62.71, 62.51; H = 7.70, 7.76; N = 10.91, 10.90; Cl = 9.21, 9.37.

Example 6

4-Acetylamino-5-chloro-2-methoxy-N-[3-β-(9-neopentyl-9-azabicyclo (3,3,1)nonyl]benzamide (E6)

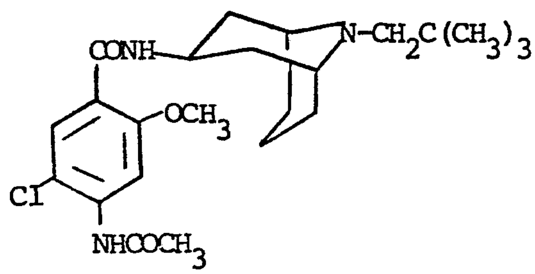

(E6)

4-Acetylamino-5-chloro-2-methoxy benzoic acid (6.57g, 0.027 mole) was treated with oxalyl chloride (2.50ml), triethylamine (11.2 ml) and 3-β-amino-9-neopentyl-9-azabicyclo(3,2,1)nonane (D4) (5.67g, 0.27 mole) in a similar manner to that described in Example 1 give the title compound as colourless microcrystals m.p. 197-198°.

$C_{23}H_{34}N_3O_3Cl$

Required % C=63.36; H=7.86; N=9.64; Cl=8.13

Found % C=63.20; H=7.88; N=9.47; Cl=7.61.

Example 7

4-Amino-5-bromo-2-methoxy-N-[3-β-(8-neopentyl-8-azabicyclo (3,2,1)octyl)]benzamide (E7)

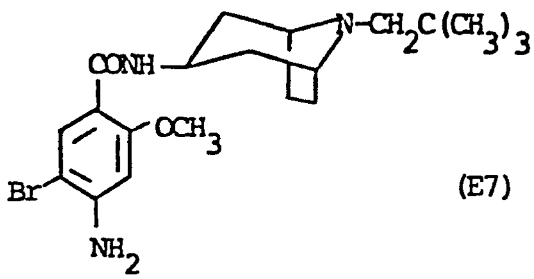

(E7)

4-Acetylamino-5-bromo-2-methoxy benzoic acid (6.57g, 0.027 mole) was treated with oxalyl chloride (2.50ml), triethylamine (11.2 ml) and 3-β-amino-8-neopentyl-8-azabicyclo (3,2,1) octane (D3)(5.67g, 0.27 mole) in a similar manner to that described in Example 1 followed by aqueous ethanolic hydrolysis to give the title compound as colourless crystals m.p. 241-243°.

$C_{20}H_{30}BrN_3O_2$

Required % C = 56.60; H=7.07; N=9.90; Br=18.87

Found %     C = 56.58; H=7.18; N=9.75; Br=18.60.

## PHARMACOLOGICAL DATA

### (i) Anti-5HT Activity

Compounds of the invention inhibit the behavioural symptoms induced by 5-methoxy-N,N-dimethyl tryptamine, a central 5-hydroxytryptamine agonist, and are central 5HT antagonists. As such they would be expected to possess anti-depressant, (Ogren, S O, Furce, K Agnati, L F, Gustofoson J A, Jonsson, G, and Holan A C, 1979, J Neural Erous, 46, 85-103) and/or anxiolytic (Stein, L, Wline, D, and Bellugi, J D, 1975, in Advance-Biochemical Psychopharmocology, ed Corta, E, and Greengord, P, Vol 14, 29-44, Rouen Press, NY) activity.

Method

Mice (♂ $CD^{-1}$ Charles Rine) are pretreated with the compounds 10 animals/group under investigation and 1 h later one group is injected with 10 mg/kg ip 5-methoxy-N,N-dimethyltryptamine (Sigma). The symptoms of fore-paw tapping movements, head jerks and splayed limbs are scored: 1, present; 0, absent, giving a maximum score of 3/mouse or 30/group. Results are expressed as the percentage inhibition compared to the group treated with 5-methoxy-N,N-dimethyl tryptamine alone. The dose of compound inhibiting the symptoms by 50% is determined graphically.

The results are shown in Table 1.

TABLE I

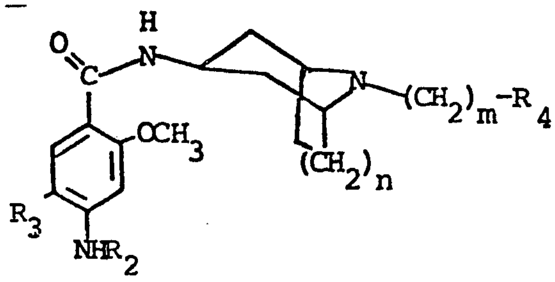

Anti 5-HT Activity

| | | | | | Inhibition of 5-MDMT induced symptoms mg/kg p.o. | |
|---|---|---|---|---|---|---|
| Example | n | $R_2$ | $R_3$ | $R_4$ | Mouse | Rat |
| 1 | 1 | $COCH_3$ | Cl | $C(CH_3)_2$ | $ED_{50}$2.4 | $ED_{50}$10 |
| 2 | 1 | H | Cl | $C(CH_3)_2$ | $ED_{50}$0.2 | $ED_{50}$7.2 |
| 3 | 1 | H | Cl | 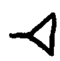 | $ED_{50}$3 | 56% at 10 |
| 4 | 2 | H | Cl | $C(CH_3)_3$ | $ED_{50}$4.6 | $ED_{50}$ 6.2 |
| 5 | 1 | H | Cl | $CH_2CH(CH_3)_2$ | $ED_{50}$8 | |
| 6 | 2 | $COCH_3$ | Cl | $C(CH_3)_3$ | - | $ED_{50}$ 10 |
| 7 | 1 | H | Br | $C(CH_3)_3$ | $ED_{50}$ 4 | |

(ii) Dopamine Receptor Blocking Activity in the CNS

The test is based on that described by Protais, P., Constantin, J. and Schwartz J.C. (1976), Psychopharmacology, 50, 1-6.

When mice are given a dose of 1 mg/kg apomorphine and then placed in an enclosed environment, such as an inverted wire cage, they are seen to climb around the walls. This behavioural phenomenon is thought to be a consequence of the stimulation of post-synaptic Dopamine (D.A.) receptors in the nucleus accumbens. Inhibition of apomorphine induced climbing is therefore indicative of post-synaptic D.A. receptor blockade in the accumbens.

Groups of 10 male CD1 mice, weighing 25-30 g were pre-treated orally with either graded doses of the test compound or vehicle, at appropriate time intervals before the subcutaneous administration of a sub-maximal dose of apomorphine (1 mg/kg). Immediately after the apomorphine injection the mice were placed in wire 'climbing cages' and each animal was scored for climbing behavour at 10 and 20 minutes post apomorphine as follows:-

Four paws on cage floor = 0
Fore paws on cage wall = 1
Four paws on cage wall = 2

The total score was calculated for each group of mice and expressed as a percentage inhibition of climbing.

$$\% \text{ inhibition} = 100 - \frac{\text{Total score for test compound}}{\text{Total score for apomorphine control}}$$

ED50's and fiducial limits were calculated according to the method of Litchfield and Wilcoxon, the ED50 being the dose that produced a 50% inhibition of apomorphine - induced climbing.

The compound of Example 1 had an $ED_{50}$ of 4 mg/kg p.o.

Toxicity

No toxic effects were observed in the above tests.

Claims

1. A compound of formula (I) or a pharmaceutically acceptable salt and/or solvate thereof:

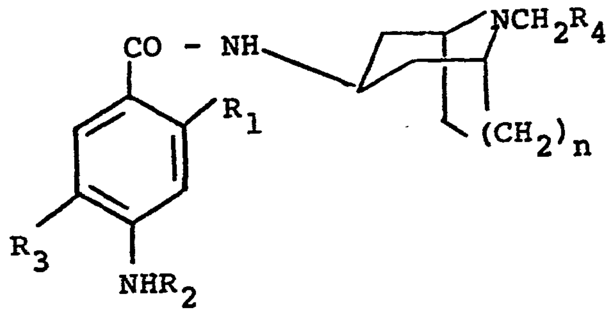

(I)

wherein:

$R_1$ is $C_{1-6}$ alkoxy;
$R_2$ is hydrogen or $C_{1-4}$ alkanoyl;
$R_3$ is chloro or bromo;
$R_4$ is t-butyl, iso-butyl or cyclopropyl; and
n is 1 or 2.

2. A compound according to claim 1 wherein $R_1$ is methoxy.

3. A compound according to claim 1 of formula (II):

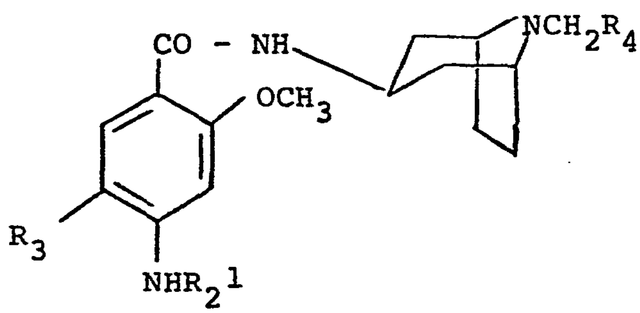

(II)

wherein $R_2^1$ is hydrogen or acetyl and $R_4$ is as defined in claim 1.

4. 4-Amino-5-chloro-2-methoxy-N-[3-β-(8-neopentyl-8-azabicyclo(3,2,1)octyl)] benzamide, 4-Amino-5-bromo-2-methoxy-N-[3-β-(8-neopentyl-8-azabicyclo (3,2,1) octyl)]benzamide, 4-amino-5-chloro-2-

methoxy-N-[3-β-(8-isopentyl-8-azabicyclo(3,2,1)octyl)] benzamide or 4-amino-5-chloro-2-methoxy-N-[3-β-(8-cyclopropylmethyl-8-azabicyclo(3,2,1)octyl] benzamide.

5. A compound according to claim 1 of formula (III):

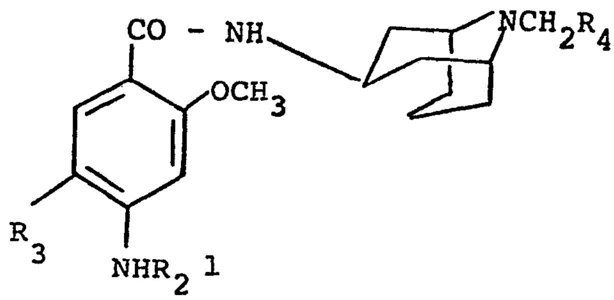

(III)

wherein $R_2^1$ and $R_4$ are as defined in claim 4.

6. A compound according to any one of claims 1,2,3 or 5 wherein $R_3$ is chloro

7. 4-Amino-5-chloro-2-methoxy-N-[3-β-(9-neopentyl-9-azabicyclo(3,3,1)nonyl)] benzamide.

8. A process for the preparation of a compound according to any one of claims 1 to 7 which process comprises the reaction of a compound of the formula (IV):

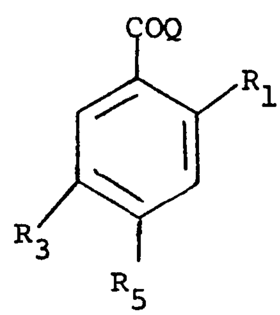

(IV)

with a compound of formula (V):

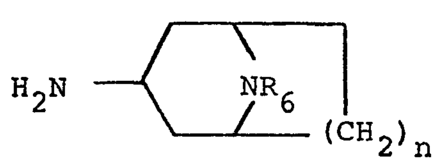

(V)

wherein:

Q is a leaving group;

$R_5$ is amino, protected amino or nitro;

$R_6$ is $CH_2R_4$ as defined in claim 1 or a hydrogenolysable protecting group; and the remaining variables are as defined in claim 1; and thereafter if necessary converting group $R_5$ in the thus formed compound to a group $NHR_2$, converting $R_6$ when other than $CH_2R_4$ and optionally forming a pharmaceutically acceptable salt of the resultant compound of the formula (I).

9. A pharmaceutical composition comprising a compound according to any one of claims 1 to 7 or a pharmaceutically acceptable salt thereof and a pharmaceutically acceptable carrier.

10. A compound of formula (V) as defined in claim 8 wherein $R_6$ is $CH_2R_4$ wherein $R_4$ is t-butyl or cyclopropyl.

11. A compound according to any one of claims 1 to 7 for use in the treatment of CNS disorders related to depression or anxiety.